# EUROPEAN PATENT SPECIFICATION

(11) **EP 1 752 768 B1**
(45) Date of publication and mention of the grant of the patent: **29.12.2010**
(21) Application number: 05741615.8
(22) Date of filing: 19.05.2005
(51) Int. Cl.: G01N 33/576, G01N 33/543, G01N 33/53

(54) **METHOD OF DETECTING HEPATITIS B VIRUS**
VERFAHREN ZUM NACHWEIS VON HEPATITIS-B-VIRUS
PROCEDE DE DETECTION DU VIRUS DE L'HEPATITE B

(30) Priority: 19.05.2004 JP 2004149682
(43) Date of publication of application: 14.02.2007
(73) Proprietor: Advanced Life Science Institute, Inc., Wako-shi, Saitama 351-0112 (JP)
(72) Inventor: OHUE, Chiharu ADVANCED LIFE SCIENCE INSTITUTE, Inc, Wako-shi, Saitama 3510112 (JP); MAKI, Noboru, ADVANCED LIFE SCIENCE INSTITUTE, Inc, Wako-shi, Saitama 3510112 (JP); KIMURA, Tatsuji, ADVANCED LIFE SCIENCE INST. Inc., Wako-shi, Saitama 3510112 (JP)
(74) Representative: Kaiser, Jürgen
(86) International application number: PCT/JP2005/009158
(87) International publication number: WO 2005/111620

(56) References cited:
- EP-A1- 0 341 439
- EP-A1- 0 967 484
- EP-A1- 1 020 727
- EP-A1- 1 306 671
- EP-A1- 1 308 730
- WO-A-2004/022585
- WO-A-2005/040815
- JP-A- 11 051 940
- JP-A- 2000 105 233
- NATH N ET AL: "Stability of the recombinant hepatitis B core antigen." JOURNAL OF CLINICAL MICROBIOLOGY JUN 1992, vol. 30, no. 6, June 1992 (1992-06), pages 1617-1619, XP002454324 ISSN: 0095-1137

## Description

### TECHNICAL FIELD

The present invention relates to a method for treatment of a sample containing hepatitis B virus (hereinafter, referred to as "HBV") to detect or quantify HBV antigens in blood with high sensitivity and a method for detection or quantification of HBV antigens using said method for treatment.

### BACKGROUND ART

HBV is the virus identified for the first time as a causative virus of post-transfusion hepatitis and HBV infection occurs through blood transfusion at the time of surgery. Accordingly, for screening of blood for transfusion, it is extremely important to make diagnosis of the presence or absence of HBV infection in blood.
As diagnostic methods for this HBV infection, there is a method for detection of antibody to HBV in a sample, a method for detection of HBV antigens, or a method for detection of HBV genes.

Among these methods, the method for detection of HBV genes includes a nucleic acid amplification test (NAT) and a DNA probe test, which are currently widely used in clinical setting. Further, attention is directed to a relation between the amount of HBVDNA and the pathosis of HBV carrier by virtue of widespread use of the NAT method, and the NAT method has come to be mainly used for monitoring after treatment with an antiviral drug.

NAT methods such as PCR method and TMA method are highly sensitive methods for detecting gene fragments. However, when HBV genomic DNA is extracted from a sample, these methods require a treatment time as long as two hours in the manual method as well as include a plural process steps, and so forth, which is complicated. In addition, the complexity of this process increases chances of contamination and increases the possibility of having false positive samples. There is also a problem that technical skills are needed to obtain consistent assay values. Although the recent development of an automated instrument has allowed measures against contamination to be taken and the processing time for DNA extraction to be shortened, a stillhighly expensive instrument is required, and thus, is not in common use except in institutions where a large number of samples are handled. Further, since DNA primers must match the target gene, several kinds of primers need to be used, which gives rise to a problem that the cost per test becomes high as compared with that of immunoassays.

Because of these problems associated with the test for the detection of HBV genome, attention is paid to methods for detection of viral antigens. In HBV antigen tests, a method for detection of HBs antigen has been conventionally used for blood screening and a method for measurment of HBe antigen has been widely used for a proliferation marker of HBV.

In addition to these antigen tests, a method for direct detection of HBV core antigen (HBc antigen) has also been developed. Usuda et al. (Journal of Virological Methods, 72, 95-103, 1998) developed a method for detection of HBc antigen in serum using monoclonal antibodies having specificity for HBV core (HBc) antigen and showed that the method was clinically useful similarly to the above-described NAT method for detection of viral genome. This HBc antigen detection system is relatively tolerant to contamination because amplification procedures are not included in the detection process.

However, there are several problems left behind even in the above method. The process of sample treatment for measurement is complicated and is time-consuming, which is problematic when the method is intended for use in screening, monitoring, and the like. For treatment of a sample (serum), a multi-step treatment process including treatment with an anti-HBs polyclonal antibody (37°C, 2 hours), centrifugation (10 min), removal of supernatant, treatment with surfactants, treatment with an alkali (35 min) and addition of a neutralizing agent, is necessary for concentration of virus particles and removal of serum components. Since these steps involve highly skilled work, certain experience is essential to obtain reproducibility. Further, a minimum treatment time of about three hours is required. Furthermore, automation is difficult and simultaneous mass treatment is also difficult because steps such as centrifugation and removal of supernatant are involved; thus in terms of the process as well, the above method is not appropriate for use that requires mass treatment.

Further, Oshihara et al. have developed a method in which HBc antigen is assayed by means of treatment with an alkali, treatment with pronase, and addition of Nonidet P40 (NP-40) that is a nonionic surfactant and mercaptoethanol without performing the treatment with anti-HBs polyclonal antibodies (Japanese Patent Laid-Open No. 8-50133). However, this method indicates low sensitivity and the concentration of HBc antigen in the detection limit is equivalent to 2.2 pg/ml of the concentration of HBV-DNA which is estimated at the order of 10⁵ to 10⁶ copies/ml.

In addition to the above-described methods for detection of HBc antigen, a method for assay of HBV core-related antigens (HBcr antigens) to allow simultaneous assay of HBe antigen and HBc antigen (International Publication WO 02/14871 A1) andamethodforassayofp22crantigenofHBV (HBVp22cr antigen) that forms an HBV virus-like particle (International Publication WO 04/22585 A1) have been developed. These methods are more sensitive than the methods for detection of HBc antigen but indicate still an unsatisfactory sensitivity when compared with the methods of measurement of HBV genome.
Further, EP-0967484-A1 discloses a method for treating a virus-containing sample, characterized by treatment of a virus containing sample with a treatment solution containing (1) an anionic surfactant and (2) an amphoteric surfactant, nonionic surfactant or protein denaturant and a method for treating a virus-containing sample with a treatment solution containing (1) a chaotropic ion and (2) an acidifying agent.

Patent Document 1: Japanese Patent Laid-Open No. 8-50133
Patent Document 2: International Publication WO 02/14871 A1
Patent Document 3: International Publication WO 04/22585 Al

Non-Patent Document 1: Journal of Virological Methods, 72, 95-103, 1998

### DISCLOSURE OF THE INVENTION

### PROBLEMS TO BE SOLVED BY THE INVENTION

Immunoassays can be performed easily and at a low cost; however, the current method for assay of HBe antigen that is used as a proliferation marker cannot measure HBe antigen occurring as immune complexes in the presence of anti-HBe antibodies. Further, the methods for assay of HBc antigen are not applied in clinical studies owing to the complexity of pretreatment as described above and insufficiency of sensitivity, although the amounts of HBc antigen correlate with the amounts of HBV DNA.

On the other hand, in the assay of HBV core-related antigens and in the assay of HBV p22cr antigen, pretreatment of a sample is carried out using a surfactant and heat (from 56 to 70°C) to disrupt antibodies and virus particles, and then HBV core-related antigens or HBV p22cr antigens are measured.
However, these methods also need to treat a sample off-board and the adaptation to the full automation is difficult.

Accordingly, the object of the present invention is to provide a pretreatment method for assay of HBV core-related antigens (HBe and HBc antigens), HBV p22cr antigen, and the like even in the presence of anti-HBV antibodies for screening of hepatitis B, monitoring in the treatment of patients with chronic hepatitis B, and so forth, and an assay method with the use thereof. The object of this disclosure is to provide a system for detection of HBV antigens that can be easily applied to a mass treatment system such as automation by simple pretreatment in shorter time.

### MEANS FOR SOLVING THE PROBLEMS

As a result of assiduous research intended to solve the above problems, the present inventors focused attention on (a) a method for treatment of a sample containing HBV that allows HBV antigens in the sample to be converted into a state suitable for detection with a probe only by a simple procedure in a short time and (b) a method for treatment that allows antibodies against HBV antigens originating from a host that compete with a probe for capture or detection to be simultaneously inactivated by the simple procedure in a short time in order to detect HBV antigens in the sample. Further, the present inventors found that, for assay of HBV antigens, not only can HBV antigens present in a sample be released from virus particles or immune complexes but also human antibodies against HBV present in the sample are inactivated by (c) treatment of the sample with an acidifying agent and (d) treatment with a surfactant, a protein denaturant, and a reducing agent in addition to the former treatment, and that (e) a sample most suitable for an immunoassay with a probe such as antibody can be provided by the use of the treatment method. Furthermore, the present inventors found it possible to provide (f) a step of treating a sample with a treatment agent that releases HBV antigens present in the sample containing HBV antigens from the virus particles and that also simultaneously inactivates the human antibodies against HBV present in the sample, a method for detection and quantification of HBV antigens by an immunoassay involving the treatment step, and (g) an HBV antigen assay kit containing the treatment agent, and achieved the present invention based on these findings.

Thus, the following items 1 to 3 shown below are provided according to the present invention:
1. A method for treatment of a sample containing HBV **characterized in that** release of HBV antigens and inactivation of antibodies that bind to HBV antigens are performed by treating the sample, containing hepatitis B virus with a treatment agent, containing:
   (1) an acidifying agent selected from the group of hydrochloric acid, sulfuric acid, trifluoroacetic acid and trichloroacetic acid, and
   (2) a cationic surfactant having an alkyl group of 12 or more carbon atoms and a tertiary amine or a quaternary ammonium salt within the same molecule.
2. A method for immunological detection of HBV antigens including
   (1) a step of conducting the treatment of a sample containing hepatitis B virus according to the preceding item 1 and
   (2) a step of detecting hepatitis B virus antigens with the use of a probe that binds to the hepatitis B virus antigens.
3. Use of a diagnostic kit for diagnosis of HBV infection, the kit comprising:
   (a) a treatment agent for treating a sample containing HBV, comprising:
      (1) an acidifying agent selected from the group of hydrochloric acid, sulfuric acid, trifluoroacetic acid and trichloroacetic acid, and
      (2) a cationic surfactant having an alkyl group of 12 or more carbon atoms and a tertiary amine or a quaternary ammonium salt within the same molecule.
   (b) a probe that binds to an hepatitis B virus antigen.

### ADVANTAGES OF THE INVENTION

According to the present invention, it becomes possible to release HBV antigens easily in a short time from the virus particles in a state suitable for an immunoassay method in which an antigen is detected with a probe such as antibody as well as to inactivate antibodies against HBV antigens. Further, it becomes possible to detect and quantify HBV antigens easily in a short time with high sensitivity by treating a sample containing HBV according to the method described in the present invention and subjecting to the immunoassay method in which an antigen is detected with a probe such as antibody. Furthermore, according to the present invention, it is possible to solve the problem of precipitation caused by acid treatment by the use of the surfactant and the like in addition to the acidifying agent, disrupt protein efficiently, release viral antigens with ease in a short time, and bring about a remarkably excellent sensitivity-enhancing effect.

### BRIEF DESCRIPTION OF THE DRAWINGS

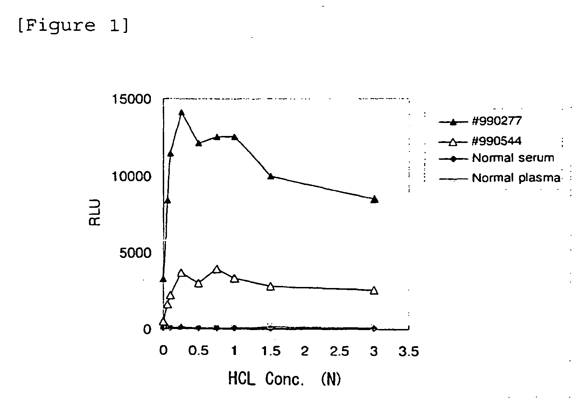

Figure 1 is a graph showing the result of the effect depending on the concentration of an acidifying agent (hydrochloric acid) in sample treatment.

### DESCRIPTION OF THE SYMBOLS

-▲- HBV Antigen Positive Sample (#990277)
-Δ- HBV Antigen Positive Sample (#990544)
-◆- HBV Antigen Negative Sample (Normal Serum)
-□- HBV Antigen Negative Sample (Normal Plasma)

### BEST MODE FOR CARRYING OUT THE INVENTION

Samples used in the method for treatment of a sample containing HBV antigens according to the present invention include biological fluids such as whole blood, plasma, serum, urine, saliva, and cerebrospinal fluid, liver tissues, and the like.

The infectious particle of HBV is thought to be Dane particle having a structure with a diameter of 42 nm. The envelope lipoproteins are HBs antigens, and HBc antigens form an inner nucleocapsid (core particle) with a diameter of 27 nm. In addition, there is HBV p22cr antigen that forms an HBV nucleocapsid-like particle, and this molecule is thought to form a core-like particle and have HBs antigen on its outside.

The diagnosis of hepatitis B is generally performed by detecting HBs antigen or HBe antigen. However, the measurement of these antigens do not accurately reflect the time of the infection and the amount of infectious particles. For this reason, it is necessary to determine HBc antigen, HBV core-related antigens, and HBV p22cr antigen that make up the virus particle or the virus-like particle.

In samples, HBc antigen and p22cr antigen form the virus particle and HBe antigen and the like forms immune complexes with anti-HBV antibodies. In order to detect HBc antigen, HBe antigen, and HBV p22cr antigen among these antigens, it is necessary to I) allow HBc antigen and HBV p22cr antigen not only to be released from HBV particles by disrupting the HBV particles but also to be converted to their monomer forms as much as possible, II) inactivate or remove antibodies, originating from a host, against HBc antigen and HBe antigen of HBV, and III) release HBc antigen, HBe antigen, and HBV p22cr antigen from interactions with blood components other than the antibodies against the HBV antigens. Although the antibodies against the HBV antigens can be removed by centrifugation and affinity chromatography, treatment steps increase, and therefore, it seems desirable to carry out the inactivation.

A maximum release of HBc antigen, HBe antigen, and HBV p22cr antigen, contained in a limited amount of sample in a detection system, in their monomer states from HBV particles, antibodies against HBV antigens, and other blood components results in an increase of the number of the antigen molecules that can react with a probe. It is important to maximally release the antigens in their monomer states by a short-time and simple sample treatment, thereby enhancing their reactivity with a probe.

As the conditions to inactivate the activities of antibodies present in a sample, an alkaline treatment, an acid treatment, and the like are known. When serum and the like are subjected to an acid treatment, certain serum-derived proteins and the like are irreversibly denatured and precipitation or cloudiness occurs in certain cases. Therefore, when a sample after treated with an acid is pipetted, trouble such as clogging often occurs. Further, in measurement, precipitates entangling denatured proteins and the like may adsorb to a carrier or solid phase linked with a probe such as antibody to capture a target antigen, resulting in a false positive. In addition, the target antigen is entangled in those precipitates and the amount of the antigen that can be bound to the probe is decreased, thereby presenting a problem of sensitivity reduction.

The present invention makes it possible to achieve prevention of precipitation and cloudiness caused by the acid treatment, prevention of false positive, and enhancement of sensitivity by adding another substance to an acidifying agent.
Here, as the acidifying agent, hydrochloric acid, sulfuric acid, acetic acid, trifluoroacetic acid, trichloroacetic acid are appropriate. In particular, the concentration of the acidifying agent at the treatment is preferably 0.05 N or higher and 1 N or lower, and further preferably from 0.25 N to 1 N. In this case, a sample added acidifying agent is treated at pH 2.5 or lower, and in most samples at pH 2.0 or lower.

One of the substances added to the acidifying agent in the treatment agent includes a surfactant. Various surfactants are known to have an activity to disrupt a higher structure of protein and exert effects such as disruption of viral particle membrane, denaturation of antibodies, and solubilization of insoluble proteins. However, in the presence of such a surfactant, a conformational epitope of a target antigen is also disrupted, resulting in weakening of binding to a probe such as antibody to capture the antigen, which presents a serious problem of sensitivity reduction.

On the other hand, the denaturing activity of the surfactant may of ten be reversible, and a temporarily denatured structure is sometimes returned to the original structure by reducing the concentration of the surfactant by means of dilution or dialysis. Therefore, the antibodies originating from a sample may compete with a probe for measurement, and as the result, it is apparent that sensitivity may be reduced. Thus, the addition of the surfactant has such an ambivalent nature described above. Surfactants are classified into various groups according to their structures and properties. For example, there are ionic and nonionic surfactant, and the ionic surfactant further include anionic, cationic, amphoteric surfactants, and the like.

The present inventors have found that the problem associated with the acid treatment such as occurrence of precipitates and the problem associated with the surfactant treatment such as reactivation of antibodies in a sample can be solved by combining the acidifying agent with the cationic surfactant, and that the combination shows a significant enhancement effect in sensitivity with respect to the detection of HBV antigens.
Particularly among surfactants, the present inventors have found that a striking effect is obtained by using a cationic surfactant having a straight chain alkyl group of 12 or more carbon atoms and a tertiary amine or a quaternary ammonium salt within the same molecule.

Furthermore, it has been found that the addition of a nonionic surfactant, e.g. polyoxyethylene iso- octylphenyl ethers such as Triton X-100 or polyoxyethylene sorbitan alkyl esters such as Tween 20, the addition of a protein denaturant such as urea or thiourea, and the addition of a reducing agent such as cysteine, cysteamine, dimethyl- aminoethanethiol, diethylaminoethanethiol, diisopropyl- aminoethanethiol, or dithiothreitol to the treatment agent containing the acidifying agent and the surfactant are more preferable.

The present disclosure provides the method for treatment of a sample containing HBV **characterized in that** release of HBV antigens and inactivation of antibodies bound to HBV antigens are carried out by treating a sample containing HBV with a treatment agent containing an acidifying agent selected from the group of hydrochloric acid, sulfuric acid, trifluoroacetic acid and trichloroacetic acid, and a cationic surfactant having an alkyl group and a tertiary amine or a quaternary ammonium salt within the same molecule, a nonionic surfactant, and further a protein denaturant, and a reducing agent.

As the amphoteric surfactant having an alkyl group and a tertiary amine or a quaternary ammonium salt within the same molecule, N-dodecyl-N,N-dimethyl-3-ammonio-1-propanesulfonate, N-tetradecyl-N,N-dimethyl-3-ammonio-1-propanesulfonate, N-hexadecyl-N,N-dimethyl-3-ammonio-1-propanesulfonate, N-octadecyl-N,N-dimethyl-3-ammonio-1-propanesulfonate, and the like are appropriate.

Further, as the cationic surfactant having an alkyl group and a tertiary amine or a quaternary ammonium salt within the same molecule, decyltrimethylammonium chloride, dodecyltrimethylammonium chloride, tetradecyltrimethylammonium chloride, hexadecyltrimethylammonium chloride, decyltrimethylammonium bromide, dodecyltrimethylammonium bromide, tetradecyltrimethylammonium bromide, hexadecyltrimethylammonium bromide, lauryl pyridinium chloride, tetradecyl pyridinium chloride, cetyl pyridinium chloride, and the like are appropriate.

The concentration at the treatment of such an cationic surfactant having an alkyl group and a tertiary amine or a quaternary ammonium salt within the same molecule is preferably 0.1% or higher and 15% or lower, further preferably from 0.5% to 10%.
As the nonionic surfactant added to the acidifying agent and the cationic surfactant having an alkyl group and a tertiary amine or a quaternary ammonium salt within the same molecule, polyoxyethylene isooctylphenyl ethers such as Triton X-100, polyoxyethylene nonylphenyl ethers such as NP40 or polyoxyethylene sorbitan alkyl esters such as Tween 80 are appropriate, and their concentrations at the treatment are preferably 1% or higher and 7.5% or lower, further preferably 1% or higher and 5% or lower.

As the protein denaturant added to the acidifying agent and the cationic surfactant having an alkyl group and a tertiary amine or a quaternary ammonium salt within the same molecule, urea, thiourea, and the like are appropriate, and their concentrations at the treatment are preferably 0.5 M or higher, further preferably 1 M or higher and 8 M or lower. However, in the case of no problem of solubility, for example, when urea is added in powder form in advance in a tube for treating a sample, it is possible to use at concentrations up to 10 M.

As the reducing agent added to the acidifying agent and the cationic surfactant having an alkyl group and a tertiary amine or a quaternary ammonium salt within the same molecule, cysteine, cysteamine, dimethylaminoethanethiol, diethylaminoethanethiol, diisopropylaminoethanethiol, dithiothreitol, and the like are appropriate, and their concentrations at the treatment are preferably 0.25 mM or higher and 1000 mM or lower, further preferably 1.5 mM or higher and 200 mM or lower.

As described above, an additional substance added to the treatment agent includes a protein denaturant such as urea. Such a protein denaturant is known to have an activity to partially disrupt protein conformation by weakening hydrogen bonds, and it can disrupt viral particle membrane and denature antibodies against a target antigen in a sample. It has also an effect of solubilizing insoluble precipitates, for example, solubilizing a recombinant protein expressed in *E. coli* from its inclusion body that is an insoluble fraction. In the presence of a protein denaturant such as urea, however, a conformational epitope of the target antigen is also disrupted, resulting in weakening of binding to a probe such as antibody to capture the antigen, which presents a problem of sensitivity reduction.

On the other hand, the denaturing activity of the protein denaturant such as urea may often be reversible, and a temporarily denatured structure is sometimes returned to the original structure by reducing the concentration of the protein denaturant by means of dilution or dialysis. This results in a state in which antibodies originating from a sample may compete with a probe for measurement, and as the result, it is apparent that sensitivity may be reduced. Thus the addition of the protein denaturant such as urea has such an ambivalent nature described above.
The present inventors have perfected another treatment by finding that the problem associated with the acid treatment such as occurrence of precipitates and the problem associated with the protein denaturant treatment such as reactivation of antibodies in a sample can be solved by combining the acid treatment with the protein denaturant treatment.

The present inventors have found that the formation of precipitates by the acid treatment can be significantly decreased by adding urea, one of protein denaturants, at 1 M or higher concentration at the treatment. For this protein denaturant, urea, thiourea, and the like are appropriate. Further, the concentration of the protein denaturant at the treatment is preferably 1 M or higher, further preferably 1.5 M or higher and 8 M or lower. Furthermore, the present inventors have found that the addition of a nonionic surfactant, e. g. polyoxyethylene isooctylphenyl ethers such as Triton X100 and polyoxyethylene sorbitan alkyl esters such as Tween 20, to the treatment agent containing the acidifying agent and the protein denaturant exerts an effect on enhancement of sensitivity. In addition, it is possible to add a reducing agent to the treatment agent containing the acidifying agent and the protein denaturant.

In summary of the above, the present invention provides a method for treatment of a sample containing hepatitis B virus (HBV) **characterized in that** release of HBV antigens and inactivation of antibodies bound to HBV antigens are carried out by treating a sample containing HBV with a treatment agent containing (1) an acidifying agent selected from the group of hydrochloric acid, sulfuric acid, trifluoroacetic acid and trichloroacetic acid, and (2) cationic surfactant having an alkyl group of 12 or more carbon atoms and a tertiary amine or a quaternary ammonium salt within the same molecule.

Further, the treatment temperature in the method for treatment of a sample containing HBV according to the present invention may be high, but preferably from 20°C to 50°C, further preferably from 25°C to 42°C.
The treatment agent combined with the acidifying agent in the present invention is the cationic surfactant having an alkyl group of 12 or more carbon atoms and a tertiary amine or a quaternary ammonium salt within the same molecule. As a treatment agent combined with the acidifying agent, there is, for example, the amphoteric surfactant having an alkyl group and a tertiary amine or a quaternary ammonium salt within the same molecule, the cationic surfactant having an alkyl group and a tertiary amine or a quaternary ammonium salt within the same molecule, the protein denaturant, the nonionic surfactant, the reducing agent, or an anionic surfactant. By combining two or more of these treatment agents and treating simultaneously with the acidifying agent, it is possible to efficiently treat a sample containing HBV.

The method for immunological detection of HBV antigens according to the present invention comprises the steps of releasing HBV antigens and inactivating antibodies that are binding to HBV antigens by allowing HBV-containing samples to come in contact with the treatment agent containing the acidifying agent and the surfactant according to any one of claims 1 to 5 (step 1) and detecting the HBV antigens with the use of a probe that binds to the HBV antigens (step 2).
In the step 2, as the probe used for the detection, for example, an antibody that specifically binds to an HBV antigen, any molecule that exhibits a high affinity for HBV antign can be used. It is desirable that one of the probes to capture HBV core-related antigens in a sample that has been treated in the step 1 is, for example, a monoclonal antibody such as HB44, HB114, or HB61.

The probe referred herein is, for example, a polyclonal antibody obtained by immunizing an experimental animal such as mouse, rat, guinea pig, rabbit, chicken, goat, sheep, or bovine, a monoclonal antibody produced by a hybridoma that is obtained by fusing the spleen cells and the like isolated from an immunized individual and myeloma cells or a monoclonal antibody produced by a cell line that is obtained by immortalizing spleen cells from an immunized individual or leukocytes in the blood using EB virus, a polyclonal antibody produced by human, chimpanzee, or the like that is infected with HBV, or a molecule exhibiting high specificity and affinity to HBV antigen that is produced by recombinant technology from a variable region gene fragment obtained from a cDNA or a chromosomal DNA of immunoglobulin of mouse, human, or the like, or a variable region gene fragment constructed by combining part of cDNA or chromosomal DNA of immunoglobulin, with an artificially prepared sequence.

In the method for immunological detection of HBV antigens according to the present invention, an HBV antigen forms an immune complex with the monoclonal antibody as described above by an antigen-antibody reaction. This immune complex is formed by a sandwich immunoassay system using two or more kinds of antibodies. The presence of the HBV antigen can be detected as a signal by a color development method or a chemiluminescence method using a labeling enzyme present in this immune complex. In addition, by directly binding an antibody with a fluorescent substance and so forth and allowing the fluorescent substance to be incorporated into an immune complex, HBV antigen can also be detected as a signal of the fluorescence.
Furthermore, the present invention provides the use of a kit for diagnosis of HBV infection using the above immunological detection method. This diagnosis kit contains (a) a treatment agent for treating a sample containing HBV, comprising (1) an acidifying agent selected from the group of hydrochloric acid, sulfuric acid, trifluoroacetic acid and trichloroacetic acid, and (2) a cationic surfactant having an alkyl group of 12 or more carbon atoms and a tertiary amine or a quaternary ammonium salt within the same molecule and (b) a probe such as antibody that binds to an HBV antigen.

### EXAMPLES

Hereinafter, the present invention is more specifically explained by means of the following Examples.

### Example 1

Concentration of acidifying agent: To 100 µl of an HBV antigen-negative sample or each of HBV antigen-positive samples (#990277, #990544), 100 µl each of aqueous hydrochloric acid at various concentrations was added and the mixture was incubated for 10 min at room temperature. Then 100 µl of the mixture as samples for the assay was examined in the measurement method described below.
To a 96-well microplate (FluoroNunc Module, Maxisoap surface), 100 µl of a mixture of monoclonal antibodies against HBV core-related antigens (HB44, HB114, and HB61 were mixed in a ratio of two to one to one) at a concentration of 4 µg/ml were added to each well and the plate was incubated overnight at 4°C.

After washing twice with 10 mM phosphate buffer, pH 7.3, containing 0.15 M NaCl, 350 µl of 10 mM phosphate buffer, pH 7.1, containing 0.5% casein sodium was added to each well and the plate was incubated for two hours. After removing the blocking solution, 100 µl of a reaction buffer containing a neutralizing agent and the each test sample obtained by sample treatment methods were added to each well and the plate was incubated for two hours at room temperature with shaking, washed six times with 350 µl of 10 mM phosphate buffer, pH 7.3, containing 0.05% Tween 20 (washing solution), and then 100 µl of alkaline phosphatase (ALP)-labeled monoclonal antibodies (HB91 and HB110 were mixed in equal amounts)was added to each well, and the plate was incubated for 30 min at room temperature. After washing six times with the washing solution, 100 µl of a substrate solution (TROPIX, CDP-star with Emerald II) was added and the plate was incubated for 20 min.

Luminescence intensity was measured with a luminometer (DIA-IATRON, Luminous CT-9000D) and the result is shown in Figure 1. It should be noted that the concentration of hydrochloric acid shown in Figure 1 is represented by the concentration at the treatment after mixing a sample with a treatment agent.
Immunoreactivity of HBV core-related antigens could hardly be detected in HBV antigen-positive samples (#990277, #990544) incubated in a solution not containing hydrochloric acid for 10 min at room temperature. However, the immunoreactivity of HBV core-related antigens started to be observed when the concentration of hydrochloric acid at the treatment was 0.05 N or higher and reached a peak at from 0.25 to 1.0 N. Further, when the study was carried out using sulfuric acid in place of hydrochloric acid, almost the same result was obtained.

### Example 2

Concentrations of various surfactants in the presence of acidifying agent: To 100 µl of the HBV antigen-negative sample or each of the HBV antigen-positive sample (#990277, #990544, #990768), 100 µl of various surfactants dissolved in 1.ON aqueous hydrochloric acid was added and the mixture was incubated for 10 min at room temperature. 100 µl of the treated sample was used for the assay, and was subjected to examination in the method described in Example 1. The results are shown in Tables 1 to 5. In each table, the underlined portions of the measurement values indicate cases exceeding each judgment criterion.

According to Tables 1 to 5, a surfactant that showed reactivity higher than the criterion for each sample in at least one sample of the three samples was judged to have an effect to detdct HBV core-related antigen sensitively. As the result, when various surfactants were added together with an acidifying agent such as hydrochloric acid or sulfuric acid, surfactants that greatly enhanced immunoreactivity of HBV core-related antigens in the HBV antigen-positive samples were found. The surfactants for which the effects of addition were observed were amphoteric surfactants having an alkyl group and a tertiary amine or a quaternary ammonium salt within the same molecule which are not part of the invention and cationic surfactants having an alkyl group and a tertiary amine or a quaternary ammonium salt within the same molecule.

Further, the effects of addition were also found in nonionic surfactants such as Triton X100 and Tween 20. Although anionic surfactants which are not part of the invention, sodium dodecyl sulfate (SDS) and lithium dodecyl sulfate (LDS), at a concentration equal to or higher than 0.5% produced cloudiness during reaction with the samples, their effects could be confirmed by dissolving after addition of the reaction buffer containing the neutralizing agent. A surfactant having a steroid skeleton such as CHAPS did not indicate an enhancement in reactivity. In addition, sodium N-lauroyl sarcosine, deoxycholic acid, and the like were examined, but their solubility was not sufficient in the presence of the acidifying agent.

An increase in the sensitivity was observed by adding to the acidifying agent an amphoteric surfactant having an alkyl group and a tertiary amine or a quaternary ammonium salt within the same molecule which is not part of the invention or a cationic surfactant having an alkyl group and a tertiary amine or a quaternary ammonium salt within the same molecule. When the acidifying agent was removed from this treatment agent consisting of the acidifying agent and the surfactant and the samples was treated only with the surfactant that was found to be effective, the sensitivity was significantly reduced. Hence, it was considered that enhancement in the sensitivity was based on the acidifying agent, and that the sensitivity was significantly increased by adding surfactants to the acidifying agent.

**[Table 1]**

| TAC series | | | | | |
|---|---|---|---|---|---|
| | | HBV-negative sample | HBV-positive sample | | |
| | | Normal serum | #990277 | #990544 | #990768 |
| | Conc.(%) | | | | |
| No addition | 0 | 139 | 9083 | 2838 | 37054 |
| Criterion of surfactant effect | | | 13625 | 4257 | 55581 |

| | | | | | |
|---|---|---|---|---|---|
| Surfactant added to 0.5N HCl | | | | | |
| | | | | | |
| Octyltrimethylammonium Chloride | 0.5 | 110 | 8776 | 2594 | 25449 |
| [CH₃(CH₂)₇N(CH₃)₃]Cl | 1 | 104 | 6810 | 2161 | 18317 |
| | 2 | 63 | 4566 | 1457 | 11334 |
| | 5 | 81 | 1804 | 673 | 3003 |
| | | | | | |
| Decyltrimethylammmonium Chloride | 0.5 | 118 | 7977 | 2224 | 19989 |
| [CH₃(CH₂)₉N(CH₃)₃]Cl | 1 | 83 | 5914 | 2136 | 14519 |
| | 2 | 94 | 7125 | 3167 | 11655 |
| | 5 | 90 | 4351 | 1386 | 7001 |
| | | | | | |
| Dodecyltrimethylammmonium Chloride | 0.5 | 209 | 13014 | **4413** | 30270 |
| [CH₃(CH₂)₁₁N(CH₃)₃]Cl | 1 | 139 | 11494 | **5355** | 35331 |
| | 2 | 217 | **16478** | 3774 | 28240 |
| | 5 | 141 | 10287 | **7048** | 18743 |
| | | | | | |
| Tetradecyltrimethylammmonium Chloride | 0.5 | 200 | **14941** | **5985** | 37661 |
| [CH₃(CH₂)₁₃N(CH₃)₃]Cl | 1 | 208 | **15517** | **6313** | 33214 |
| | 2 | 177 | **16628** | **4997** | 35434 |
| | 5 | 224 | 12075 | 4233 | 26590 |
| | | | | | |
| Hexadecyltrimethylammonium Chloride | 0.5 | 182 | **15908** | **8062** | 42647 |
| [CH₃(CH₂)₁₅N(CH₃)₃]Cl | 1 | 198 | **14148** | **6269** | 32201 |
| | 2 | 220 | **15228** | 4207 | 30831 |
| | 5 | 260 | 12828 | 2494 | 21812 |
| | | | | | |
| Lauryl pyridinium Chloride | 0.5 | 230 | 9473 | 2510 | 24303 |
| [C₅H₅NCH₂(CH₂)₁₀CH₃]Cl | 1 | 246 | 11807 | 2335 | 20982 |
| | 2 | 217 | 10043 | 1932 | 13585 |
| | 5 | 264 | 7401 | 1970 | 14991 |

**[Table 2]**

| TAB series | | | | | |
|---|---|---|---|---|---|
| | | HBV-negative sample | HBV-positive sample | | |
| | | Normal serum | #990277 | #990544 | #990768 |
| | Conc.(%) | | | | |
| No addition | 0 | 136 | 11014 | 2634 | 34595 |
| Criterion of surfactant effect | | | 15420 | 3688 | 48433 |
| | | | | | |
| Surfactant added to 0.5N HCl | | | | | |
| | | | | | |
| Hexyltrimethylammonium Bromide | 0.5 | 253 | 12044 | **4302** | 27024 |
| [CH₃(CH₂)₅N(CH₃)₃]Br | 1 | 249 | 10241 | **4602** | 28610 |
| | 2 | 214 | 7842 | **4301** | 22250 |
| | 5 | 107 | 5165 | 3577 | 13782 |
| | | | | | |
| Octyltrimethylammonium Bromide | 0.5 | 136 | 11316 | 3528 | 32491 |
| [CH₃(CH₂)₇N(CH₃)₃]Br | 1 | 103 | 8832 | 3424 | 23472 |
| | 2 | 198 | 6652 | 3268 | 16111 |
| | 5 | 57 | 2596 | 793 | 3769 |
| | | | | | |
| Decyltrimethylammmonium Bromide | 0.5 | 146 | 10862 | 2868 | 26087 |
| [CH₃(CH₂)₉N(CH₃)₃]Br | 1 | 103 | 9975 | 3308 | 16957 |
| | 2 | 58 | 6988 | 2558 | 10411 |
| | 5 | 80 | 4466 | 2677 | 8274 |
| Dodecyltrimethylammmonium Bromide [CH₂(CH₂)₁₁N(CH₃)₃]Br | 0.5 | 165 | 14345 | 3673 | 30367 |
| | 1 | 116 | 9962 | **4581** | 29491 |
| | 2 | 216 | 15309 | **5578** | 26777 |
| | 5 | 190 | 10204 | **5402** | 17091 |
| | | | | | |
| Tetradecyltiimethylammmonium Bromide | 0.5 | 209 | **16926** | **6645** | 43841 |
| [CH₃(CH₂)₁₃N(CH₃)₃]Br | 1 | 278 | **16655** | **6869** | 37064 |
| | 2 | 401 | 14375 | **6827** | 29279 |
| | 5 | 294 | 13023 | 3300 | 24118 |
| | | | | | |
| Hexadecyltrimethylammonium Bromide | 0.5 | 227 | **18706** | **8451** | **56923** |
| [CH₃(CH₂)₁₅N(CH₃)₃]Br | 1 | 254 | **19937** | **7813** | 35855 |
| | 2 | 244 | 15221 | **4128** | 27210 |
| | 5 | 602 | 11052 | 2539 | 17021 |

**[Table 3]**

| APS series | | | | | |
|---|---|---|---|---|---|
| | | HBV-negative sample | HBV-positive sample | | |
| | | Normal serum | #990277 | #990544 | #990768 |
| | Conc.(%) | | | | |
| No addition | 0 | 148 | 9294 | 2175 | 38028 |
| Criterion of surfactant effect | | | 13941 | 3263 | 57042 |
| | | | | | |
| Surfactant added to 0.5N HCl | | | | | |
| | | | | | |
| 3-[3-(Cholamidopropyl)dimethyl-ammonio]-1-propanesulfonate | 0.5 | 192 | 10068 | 2306 | 28850 |
| | 1 | 185 | 9429 | 1498 | 19808 |
| | 2 | 185 | 7823 | 1205 | 17336 |
| | 5 | 122 | 6911 | 881 | 14768 |
| | | | | | |
| N-Dodecyl-N, N-dimethyl-3-ammonio-1-propanesulfonate | 0.5 | 171 | **17118** | **5251** | **43825** |
| | 1 | 170 | **26990** | **7945** | **50648** |
| CH₃(CH₂)₁₁N(CH₃)₂[(CH₂)₃SO₃] | 2 | 116 | **28246** | **7316** | **55167** |
| | 5 | 143 | **32885** | **9698** | **47512** |
| | | | | | |
| N-Tetradecyl-N, N-dimethyl-3-ammonio-1-propanesulfonate | 0.5 | 135 | **17307** | **5976** | **48591** |
| | 1 | 123 | **30527** | **9273** | **62242** |
| CH₃(CH₂)₁₃N(CH₃)₂[(CH₂)₃SO₃] | 2 | 212 | **36256** | **8276** | **66746** |
| | 5 | 121 | **42918** | **16172** | **64794** |
| | | | | | |
| N-Hexadecyl-N, N-dimethyl-3-ammonio-1-propanesulfonate | 0.5 | 158 | **26139** | **9224** | **73370** |
| | 1 | 113 | **25348** | **7818** | **85287** |
| CH₃(CH₂)₁₅N(CH₃)₂[(CH₂)₃SO₃] | 2 | 170 | **41969** | **9925** | **75342** |
| | 5 | 161 | **35782** | **11916** | 55028 |
| | | | | | |
| N-Octadecyl-N, N-dimethyl-3-ammonio-1-propanesulfonate | 0.5 | 269 | **21151** | **11844** | **67221** |
| | 1 | 206 | **20731** | **9871** | **57204** |
| CH₃(CH₂)₁₇N(CH₃)₂[(CH₂)₃SO₃] | 2 | 283 | **24829** | **13125** | **73931** |
| | 5 | 229 | **34152** | **11351** | **77719** |

**[Table 4]**

| Nonionic series | | | | | |
|---|---|---|---|---|---|
| | | HBV-negative sample | HBV-positive sample | | |
| | | Normal serum | #990277 | #990544 | #990768 |
| | Conc.(%) | | | | |
| No addition | 0 | 180 | 10750 | 2494 | 39102 |
| Criterion of surfactant effect | | | 16125 | 3741 | 58653 |
| | | | | | |
| Surfactant added to 0.5N HCl | | | | | |
| | | | | | |
| Triton X-100 | 0.5 | 233 | 14133 | 2817 | 35471 |
| | 1 | 174 | 14708 | 2871 | 33252 |
| | 2 | 181 | 14034 | 3128 | 39651 |
| | 5 | 160 | **17943** | 3504 | 38406 |
| | | | | | |
| Triton X-114 | 0.5 | 170 | 11857 | 2856 | 27651 |
| | 1 | 188 | 12349 | 2412 | 27956 |
| | 2 | 160 | 12364 | 2874 | 29669 |
| | 5 | 155 | 12854 | 2166 | 33038 |
| | | | | | |
| Tween 20 | 0.5 | 205 | 12619 | 2732 | 34325 |
| | 1 | 205 | 12781 | 2639 | 29741 |
| | 2 | 232 | **18025** | 3703 | 58099 |
| | 5 | 202 | **35451** | **8842** | **95495** |
| | | | | | |
| Tween 60 | 0.5 | 240 | 11401 | **4234** | 49473 |
| | 1 | 194 | **17189** | **4315** | **64879** |
| | 2 | 245 | **18620** | **10542** | **137707** |
| | 5 | 209 | **25964** | **7551** | **144107** |
| | | | | | |
| Tween 80 | 0.5 | 234 | 13184 | 3086 | 39029 |
| | 1 | 115 | 13639 | 3532 | 51167 |
| | 2 | 216 | **20826** | **7126** | **98056** |
| | 5 | 236 | **43646** | **15778** | **173578** |
| | | | | | |
| Bridj 35 | 0.5 | 175 | 11862 | 2797 | 34825 |
| | 1 | 197 | 10424 | 2009 | 45445 |
| | 2 | 289 | **16694** | 2912 | 43862 |
| | 5 | 259 | 7897 | 2659 | 43319 |
| | | | | | |
| MEGA-10 | 0.5 | | | | |
| | 1 | 145 | 9680 | 2793 | 29472 |
| | 2 | 253 | 14129 | **4328** | 45222 |
| | 5 | 332 | **20245** | **10125** | **85021** |

**[Table 5]**

| Anionic series | | | | | |
|---|---|---|---|---|---|
| | | HBV-negative sample | HBV-positive sample | | |
| | | Normal serum | #990277 | #990544 | #990768 |
| | Conc.(%) | | | | |
| No addition | 0 | 167 | 7793 | 2679 | 26761 |
| Criterion of surfactant effect | | | 11690 | 4019 | 40142 |
| | | | | | |
| Surfactant added to 0.5N HCl | | | | | |
| | | | | | |
| Sodium Dodecyl Sulfate | 0.5 | 160 | **12516** | 3772 | **53253** |
| CH₃(CH₂)₁₁OSO₃Na | 1 | 152 | **18948** | **5767** | **84477** |
| | 2 | 204 | **45240** | **13482** | **168004** |
| | 5 | 301 | **31229** | **16960** | **168734** |
| | | | | | |
| Lithium Dodecyl Sulfate | 0.5 | 196 | **14409** | **4239** | **53651** |
| CH₃(CH₂)₁₁OSO₃Li | 1 | 135 | **20956** | **6324** | **46040** |
| | 2 | 236 | **29248** | **10044** | **89739** |
| | 5 | 448 | **37765** | **23853** | **199795** |

### Industrial Applicability

The present invention provides a simple and highly user-friendly sample treatment method for detection or quantification of HBV antigens in blood with high sensitivity and a method for detection or quantification of HBV with the use thereof and allows diagnosis of the presence or absence of HBV infection in blood and fast and accurate screening of blood for transfusion. The present invention can also provide the use of a diagnostic kit and greatly contributes to efficiency enhancement of HBV antigen detection.

## Claims

1. A method for treatment of a sample containing hepatitis B virus (HBV) **characterized in that** release of HBV antigens and inactivation of antibodies that bind to HBV antigens are performed by treating the sample, containing HBV with a treatment agent, containing:
(1) an acidifying agent selected from the group of hydrochloric acid, sulfuric acid, trifluoroacetic acid and trichloroacetic acid, and
(2) a cationic surfactant having an alkyl group of 12 or more carbon atoms and a tertiary amine or a quaternary ammonium salt within the same molecule.

2. The method for treatment of a sample containing HBV according to claim 1, wherein a nonionic surfactant selected from the group of polyoxyethylene isooctyl-phenyl ether, polyoxyethylene nonylphenyl ether, polyoxyethylene sorbitan alkyl ester, Brij 35 polyoxyethylene dodecyl ether and MEGA-10 decanoyl-N-methylglucamide, is further added to the treatment agent.

3. The method for treatment of a sample containing HBV according to claim 1, wherein a protein denaturant selected from the group of urea and thiourea, is further added to the treatment agent.

4. The method for treatment of a sample containing HBV according to claim 1, wherein a reducing agent selected from the group consisting of cystein, cysteamine, dimethylaminoethanethiol, diethylaminoethanethiol and dithiothreitol, is further added to the treatment agent.

5. The method for treatment of a sample containing HBV according to claim 1, wherein the cationic surfactant having an alkyl group of 12 or more carbon atoms and a tertiary amine or a quaternary ammonium salt within molecule is dodecyltrimethylammonium chloride, tetradecyltrimethylammonium chloride, hexadecyltrimethylammonium chloride, dodecyltrimethylammonium bromide, tetradecyltrimethylammonium bromide, hexadecyltrimethylammonium bromide, lauryl pyridinium chloride, tetradecyl pyridinium chloride, or cetyl pyridinium chloride.

6. A method for immunological detection of HBV antigens, comprising:
(1) a step of conducting the treatment of a sample containing HBV according to any one of claims 1 to 5; and
(2) a step of detecting HBV antigens with the use of a probe that binds to the HBV antigens.

7. Use of a diagnostic kit for diagnosis of HBV infection, the kit comprising:
(a) a treatment agent for treating a sample containing HBV, comprising:
(1) an acidifying agent selected from the group of hydrochloric acid, sulfuric acid, trifluoroacetic acid and trichloroacetic acid, and
(2) a cationic surfactant having an alkyl group of 12 or more carbon atoms and a tertiary amine or a quaternary ammonium salt within the same molecule.
(b) a probe that binds to an HBV antigen.

8. The use of a diagnostic kit according to claim 7, wherein a nonionic surfactant selected from the group of polyoxyethylene isooctyl-phenyl ether, polyoxyethylene nonylphenyl ether, polyoxyethylene sorbitan alkyl ester, Brij 35 polyoxyethylene dodecyl ether and MEGA-10 decanoyl-N-methylglucamide, is further added to the treatment agent.

9. The use of a diagnostic kit according to claim 7, wherein a protein denaturant selected from the group of urea and thiourea, is further added to the treatment agent.

10. The use of a diagnostic kit according to claim 7, wherein a reducing agent selected from the group consisting of cystein, cysteamine, dimethylaminoethanethiol, diethylaminoethanethiol and dithiothreitol, is further added to the treatment agent.

11. The use of a diagnostic kit according to claim 7, wherein the cationic surfactant having an alkyl group of 12 or more carbon atoms and a tertiary amine or a quaternary ammonium salt within molecule is dodecyltrimethylammonium chloride, tetradecyltrimethylammonium chloride, hexadecyltrimethylammonium chloride, dodecyltrimethylammonium bromide, tetradecyltrimethylammonium bromide, hexadecyltrimethylammonium bromide, lauryl pyridinium chloride, tetradecyl pyridinium chloride, or cetyl pyridinium chloride.

## Patentansprüche

1. Verfahren zur Behandlung einer Probe enthaltend Hepatitis B Virus (HBV), **dadurch gekennzeichnet, dass** eine Freisetzung von HBV-Antigenen und eine Inaktivierung von Antikörpern, die an HBV-Antigene binden, erfolgt durch Behandeln der Probe enthaltend HBV mit einem Behandlungsmittel enthaltend: (1) ein Mittel zum Ansäuren ausgewählt aus der Gruppe bestehend aus Salzsäure, Schwefelsäure, Trifluoressigsäure und Trichloressigsäure, und ein kationisches Tensid mit einer Alkylgruppe mit 12 oder mehr Kohlenstoffatomen und einem tertiären Amin oder einem quartären Ammoniumsalz im selben Molekül.

2. Verfahren zur Behandlung einer Probe enthaltend HBV nach Anspruch 1, wobei weiterhin ein nichtionisches Tensid, ausgewählt aus der Gruppe bestehend aus Polyoxyethylenisooctylphenylether, Polyoxyethylennonylphenylether, Polyoxyethylensorbitanalkylester, Brij 35, Polyoxyethylendodecylether und MEGA-10 Decanoyl-N-methylglucamid zum Behandlungsmittel hinzugefügt wird.

3. Verfahren zur Behandlung einer Probe enthaltend HBV nach Anspruch 1, wobei weiterhin ein Proteindenaturierungsmittel ausgewählt aus der Gruppe bestehend aus Harnstoff und Thioharnstoff zum Behandlungsmittel hinzugefügt wird.

4. Verfahren zur Behandlung einer Probe enthaltend HBV nach Anspruch 1, wobei weiterhin ein Reduktionsmittel ausgewählt aus der Gruppe bestehend aus Cystein, Cysteamin, Dimethylaminoethanthiol, Diethylaminoethanthiol und Dithiothreitol zum Behandlungsmittel hinzugefügt wird.

5. Verfahren zur Behandlung einer Probe enthaltend HBV nach Anspruch 1, wobei das kationische Tensid mit einer Alkylgruppe mit 12 oder mehr Kohlenstoffatomen und einem tertiären Amin oder einem quartären Ammoniumsalz im selben Molekül Dodecyltrimethylammoniumchlorid, Tetradecyltrimethylammoniumchlorid, Hexadecyltrimethylammoniumchlorid, Dodecyltrimethylammoniumbromid, Tetradecyltrimethylammoniumbromid, Hexadecyltrimethylammoniumbromid, Laurylpyridiniumchlorid, Tetradecylpyridiniumchlorid oder Cetylpyridiniumchlorid ist.

6. Verfahren für den immunologischen Nachweis von HBV-Antigenen, umfassend:
(1) einen Schritt zum Durchführen der Behandlung einer Probe enthaltend HBV nach einem der Ansprüche 1 bis 5; und
(2) einen Schritt zum Nachweisen von HBV-Antigenen unter Verwendung einer Sonde, die an die HBV-Antigene bindet.

7. Verwendung eines Diagnosekits zur Diagnose einer HBV-Infektion, wobei der Kit umfasst:
a) ein Behandlungsmittel zum Behandeln einer Probe enthalten HBV, umfassend:
(1) ein Mittel zum Ansäuren ausgewählt aus der Gruppe bestehend aus Salzsäure, Schwefelsäure, Trifluoressigsäure und Trichloressigsäure, und
(2) ein kationisches Tensid mit einer Alkylgruppe mit 12 oder mehr Kohlenstoffatomen und einem tertiären Amin oder quartären Ammoniumsalz im selben Molekül,
b) eine Sonde, die an ein HBV-Antigen bindet.

8. Verwendung eines Diagnosekits nach Anspruch 7, wobei weiterhin ein nichtionisches Tensid ausgewählt aus der Gruppe bestehend aus Polyoxyethylenisooctylphenylether, Polyoxyethylennonylphenylether, Polyoxyethylensorbitanalkylester, Brij 35, Polyoxyethylendodecylether und MEGA-10 Decanoyl-N-methylglucamid zum Behandlungsmittel hinzugefügt wird.

9. Verwendung eines Diagnosekits nach Anspruch 7, wobei weiterhin ein Proteindenaturierungsmittel ausgewählt aus der Gruppe bestehend aus Harnstoff und Thioharnstoff zum Behandlungsmittel hinzugefügt wird.

10. Verwendung eines Diagnosekits nach Anspruch 7, wobei weiterhin ein Reduktionsmittel ausgewählt aus der Gruppe bestehend aus Cystein, Cysteamin, Dimethylaminoethanthiol, Diethylaminoethanthiol und Dithiothreitol zum Behandlungsmittel hinzugefügt wird.

11. Verwendung eines Diagnosekits nach Anspruch 7, wobei das kationische Tensid mit einer Alkylgruppe mit 12 oder mehr Kohlenstoffatomen und einem tertiären Amin oder einem quartären Ammoniumsalz im selben Molekül Dodecyltrimethylammoniumchlorid, Tetradecyltrimethylammoniumchlorid, Hexadecyltrimethylammoniumchlorid, Dodecyltrimethylammoniumbromid, Tetradecyltrimethylammoniumbromid, Hexadecyltrimethylammoniumbromid, Laurylpyridiniumchlorid, Tetradecylpyridiniumchlorid oder Cetylpyridiniumchlorid ist.

## Revendications

1. Procédé de traitement d'un échantillon contre le virus de l'hépatite B (VHB) **caractérisé en ce que** la libération des antigènes du VHB et l'inactivation des anticorps qui se lient aux antigènes du VHB sont réalisées par traitement de l'échantillon contenant le VHB par un agent de traitement, contenant :
(1) un agent acidifiant choisi dans le groupe de l'acide chlorhydrique, l'acide sulfurique, l'acide trifluoroacétique et l'acide trichloroacétique, et
(2) un surfactant cationique ayant un groupe alkyle de 12 atomes de carbone ou plus et une amine tertiaire ou un sel d'ammonium quaternaire au sein de la même molécule.

2. Procédé de traitement d'un échantillon contenant le VHB selon la revendication 1, dans lequel un surfactant non ionique choisi dans le groupe de l'éther de polyoxyéthylène isooctyl-phényle, l'éther de polyoxyéthylène nonyl-phényle, un ester alkylique de polyoxyéthylène sorbitane, l'éther de polyoxyéthylène dodécyle Brij 35 et le décanoyl-N-méthylglucamide MEGA-10, est en outre ajouté à l'agent de traitement.

3. Procédé de traitement d'un échantillon contenant le VHB selon la revendication 1, dans lequel un dénaturant des protéines choisi dans le groupe de l'urée et de la thiourée, est en outre ajouté à l'agent de traitement.

4. Procédé de traitement d'un échantillon contenant le VHB selon la revendication 1, dans lequel un agent de réduction choisi dans le groupe comprenant la cystéine, la cystéamine, le diméthylaminoéthanethiol, le diéthylaminoéthanethiol et le dithiothréitol, est en outre ajouté à l'agent de traitement.

5. Procédé de traitement d'un échantillon contenant le VHB selon la revendication 1, dans lequel le surfactant cationique ayant un groupe alkyle de 12 atomes de carbone ou plus et une amine tertiaire ou un sel d'ammonium quaternaire au sein de la molécule est le chlorure de dodécyltriméthyl-ammonium, le chlorure de tétradécyltriméthylammonium, le chlorure d'hexadécyltriméthylammonium, le bromure de docédcyl-triméthylammonium, le bromure de tétradécyltriméthylammonium, le bromure d'héxadécyltriméthylammonium, le chlorure de lauryl-pyridinium, le chlorure de tétradécyl-pyridinium ou le chlorure de cétyl-pyridinium.

6. Procédé de détection immunologique des antigènes du VHB, comprenant :
(1) une étape de conduction du traitement d'un échantillon contenant le VHB selon l'une quelconque des revendications 1 à 5 ; et
(2) une étape de détection des antigènes du VHB avec l'utilisation d'une sonde qui se lie aux antigènes du VHB.

7. Utilisation d'un kit diagnostique pour le diagnostic d'une infection par le VHB, le kit comprenant :
(a) un agent de traitement pour traiter un échantillon contenant le VHB, comprenant :
(1) un agent acidifiant choisi dans le groupe de l'acide chlorhydrique, l'acide sulfurique, l'acide trifluoroacétique et l'acide trichloroacétique, et
(2) un surfactant cationique ayant un groupe alkyle de 12 atomes de carbone ou plus et une amine tertiaire ou un sel d'ammonium quaternaire au sein de la même molécule,
(b) une sonde qui se lie à un antigène du VHB.

8. Utilisation d'un kit diagnostique selon la revendication 7, dans lequel un surfactant non ionique choisi dans le groupe de l'éther de polyoxyéthylène isooctyl-phényle, l'éther de polyoxyéthylène nonyl-phényle, un ester alkylique de polyoxyéthylène sorbitane, l'éther de polyoxyéthylène dodécyle Brij 35 et le décanoyl-N-méthylglucamide MEGA-10, est en outre ajouté à l'agent de traitement.

9. Utilisation d'un kit diagnostique selon la revendication 7, dans lequel un dénaturant des protéines choisi dans le groupe de l'urée et de la thiourée, est en outre ajouté à l'agent de traitement.

10. Utilisation d'un kit diagnostique selon la revendication 7, dans lequel un agent de réduction choisi dans le groupe comprenant la cystéine, la cystéamine, le diméthylaminoéthanethiol, le diéthylaminoéthanethiol et le dithiothréitol, est en outre ajouté à l'agent de traitement.

11. Utilisation d'un kit diagnostique selon la revendication 7, dans lequel le surfactant cationique ayant un groupe alkyle de 12 atomes de carbone ou plus et une amine tertiaire ou un sel d'ammonium quaternaire au sein de la molécule est le chlorure de dodécyltriméthyl-ammonium, le chlorure de tétradécyltriméthylammonium, le chlorure d'hexadécyltriméthylammonium, le bromure de docédcyl-triméthylammonium, le bromure de tétradécyltriméthylammonium, le bromure d'héxadécyltriméthylammonium, le chlorure de lauryl-pyridinium, le chlorure de tétradécyl-pyridinium ou le chlorure de cétyl-pyridinium.
